(19) Europäisches Patentamt
European Patent Office
Office européen des brevets

(11) Veröffentlichungsnummer: **0 370 953**
**A2**

# (12) EUROPÄISCHE PATENTANMELDUNG

(21) Anmeldenummer: **89810869.1**

(22) Anmeldetag: **13.11.89**

(51) Int. Cl.$^5$: **C07D 213/127, C07D 295/12, C07C 213/08, C07C 241/02, C07C 215/90, C07D 213/20**

(30) Priorität: **21.11.88 CH 4315/88**

(43) Veröffentlichungstag der Anmeldung:
**30.05.90 Patentblatt 90/22**

(84) Benannte Vertragsstaaten:
**BE CH DE ES FR GB GR IT LI**

(71) Anmelder: **CIBA-GEIGY AG**
**Klybeckstrasse 141**
**CH-4002 Basel(CH)**

(72) Erfinder: **Vincze, Janos**
**Thiersteinerrain 122**
**CH-4059 Basel(CH)**
Erfinder: **Herter, Wilfried**
**Fecampring 17**
**D-7888 Rheinfelden(DE)**

## (54) Verfahren zur Herstellung von Farbstoffzwischenprodukten.

(57) Ein Verfahren zur Herstellung von Verbindungen der Formel

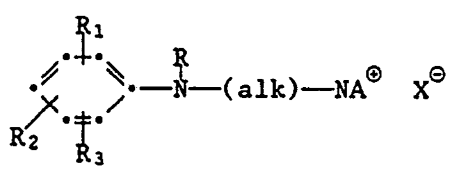

(1)

welches dadurch gekennzeichnet ist, dass man eine Verbindung der Formel

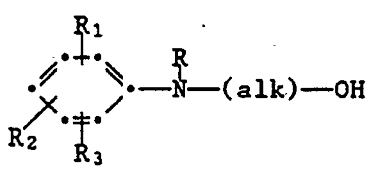

(3)

mit einem Halogenierungsmittel in Gegenwart einer Verbindung der Formel
NA (2a)
umsetzt, und, worin die Symbole die im Anspruch 1 angegebene Bedeutung haben, wird beschrieben. Die nach dem Verfahren erhältlichen Verbindungen stellen wertvolle Kupplungskomponenten für die Synthese von kationischen Farbstoffen dar.

EP 0 370 953 A2

## Verfahren zur Herstellung von Farbstoffzwischenprodukten

Die vorliegende Erfindung betrifft ein Verfahren zur Herstellung von kationischen Kupplungskomponenten sowie deren Verwendung zur Synthese von kationischen Azofarbstoffen.

Es ist bereits z.B. aus L.K.J. Tong et al., J. Am. Chem. Soc. 82, 1988-96 (1960) bekannt, N-[2-(N-Ethyl-3-methylanilino)-ethyl]-pyridiniumchlorid durch Chlorierung von N-Ethyl-N-(2-hydroxyethyl)-3-methylanilin mit Phosphoroxychlorid ($POCl_3$), Isolierung der erhaltenen Chlorethyl-Verbindung und nachfolgende Umsetzung mit Pyridin herzustellen. Dieses 2-stufige Verfahren ist umständlich und verläuft bedingt durch die Zwischenisolierung nur mit geringer Gesamtausbeute; zudem ist die Ausführung des Verfahrens im grosstechnischen Massstab wegen der starken Exothermie der Chlorierungsreaktion problematisch.

Es wurde nun überraschend gefunden, dass man entsprechende kationische Kupplungskomponenten im Eintopfverfahren mit verbesserter Ausbeute und erhöhter Verfahrenssicherheit herstellen kann.

Gegenstand der Erfindung ist daher ein Verfahren zur Herstellung von Verbindungen der Formel

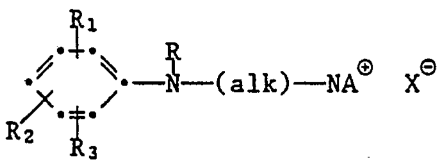 (1)

worin $R_1$, $R_2$ und $R_3$ unabhängig voneinander Wasserstoff, $C_1$-$C_4$-Alkyl, $C_1$-$C_4$-Alkoxy, Halogen, Trifluoromethyl oder $C_1$-$C_4$-Alkanoylamino bedeuten, R Wasserstoff, gegebenenfalls substituiertes $C_1$-$C_{12}$-Alkyl, oder ein Rest -(alk)-$NA^{\oplus}X^{\ominus}$ ist oder R zusammen mit $R_1$ in ortho-Stellung zur Aminogruppe einen teilhydrierten, gegebenenfalls substituierten 5- bis 7-gliedrigen heterocyclischen Ring, der gegebenenfalls noch zusätzliche N-, S- oder O-Atome als Ringglieder enthalten kann, bilden, (alk) einen gegebenenfalls substituierten $C_1$-$C_6$-Alkylenrest bedeutet, -$NA^{\oplus}$ für den Rest einer Aminoverbindung der Formel

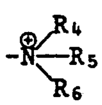 (2) oder 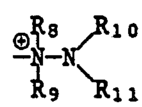 (2'),

worin $R_4$, $R_5$ und $R_6$ unabhängig voneinander gegebenenfalls substituiertes $C_1$-$C_6$-Alkyl, gegebenenfalls substituiertes $C_7$-$C_{12}$-Aralkyl oder gegebenenfalls substituiertes Aryl bedeuten, $R_8$ und $R_9$ unabhängig voneinander je gegebenenfalls substituiertes $C_1$-$C_6$-Alkyl sind und $R_{10}$ und $R_{11}$ unabhängig voneinander je für Wasserstoff, gegebenenfalls substituiertes $C_1$-$C_6$-Alkyl, gegebenenfalls substituiertes $C_7$-$C_{12}$-Aralkyl oder gegebenenfalls substituiertes Aryl stehen, oder für den positiv geladenen Rest einer 5-7 gliedrigen aliphatisch-heterocyclischen oder aromatisch-heterocyclischen Verbindung, die gegebenenfalls noch zusätzliche N-, S- oder O-Atome als Ringglieder enthält, steht, und $X^{\ominus}$ ein Halogenidion bedeutet, dadurch gekennzeichnet, dass man eine Verbindung der Formel

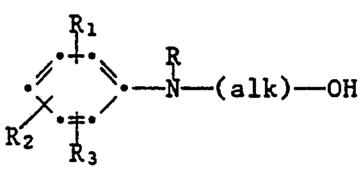 (3)

mit einem Halogenierungsmittel in Gegenwart einer Verbindung der Formel

NA (2a)

unter Freisetzung von Anionen $X^{\ominus}$ umsetzt, wobei R, $R_1$, $R_2$, $R_3$ und (alk) in der Formel (3) jeweils die oben angegebene Bedeutung haben und NA die dem Rest -$NA^{\oplus}$ zugrunde liegende ungeladene Stickstoffverbindung darstellt.

Bedeuten $R_1$, $R_2$ und/oder $R_3$ einen $C_1$-$C_4$-Alkylrest, so ist darunter generell der Methyl-, Ethyl-, n- oder iso-Propyl- oder n-, iso-, sec.-oder tert.-Butylrest zu verstehen.

Stellen $R_1$, $R_2$ und/oder $R_3$ einen $C_1$-$C_4$-Alkoxyrest dar, so kann dies generell ein Methoxy-, Ethoxy-, n-

oder iso-Propoxy- oder n-, iso-, sec.-oder tert.-Butoxyrest sein.

Stehen $R_1$, $R_2$ und/oder $R_3$ für einen Halogenrest, handelt es sich z.B. um Jod, Brom, Fluor und vorzugsweise um Chlor.

Bei $R_1$, $R_2$ oder $R_3$ als $C_1$-$C_4$-Alkanoylaminorest handelt es sich z.B. um einen Propionylamino- oder vorzugsweise um einen Acetylaminorest.

Bevorzugte Bedeutungen für die Reste $R_1$, $R_2$ und $R_3$ sind: Wasserstoff, Methyl, Ethyl, Methoxy, Ethoxy, Chlor, Acetylamino, Propionylamino.

Besonders bevorzugt bedeutet einer der Reste $R_1$, $R_2$ und $R_3$ Wasserstoff und die anderen zwei Reste unabhängig voneinander Wasserstoff, Methyl, Chlor oder Acetylamino.

In einer besonders bevorzugten Ausführungsform des erfindungsgemässen Verfahrens stehen zwei der Reste $R_1$, $R_2$ und $R_3$ für Wasserstoff und der dritte Rest für Wasserstoff, Methyl oder Chlor.

Handelt es sich bei R um einen Alkylrest, so kann dies z.B. ein Methyl-, Ethyl-, n- oder iso-Propyl, n-, sec.-, iso- oder tert.-Butylrest oder ein geradkettiger oder verzweigter Pentyl-, Hexyl-, Heptyl, Octyl-, Nonyl-, Decyl-, Undecyl-oder Dodecylrest, welcher gegebenenfalls z.B. durch $C_1$-$C_4$-Alkoxy substituiert ist, sein.

Vorzugsweise steht R für Wasserstoff oder einen unsubstituierten $C_1$-$C_6$-Alkylrest und besonders bevorzugt für Wasserstoff oder einen $C_1$-$C_4$-Alkylrest; insbesondere bevorzugte Bedeutungen von R sind Wasserstoff, Methyl und Ethyl.

Bilden R und $R_1$ gemeinsam mit dem N-Atom die Ergänzung zu einem teilhydrierten heterocyclischen Ring, so führt dies z.B. zu einem an den Benzolring ankondensierten 5- bis 7-gliedrigen, ein oder zwei N-Atome bzw. ein N- und ein S- oder O-Atom enthaltenden Heterocyclus, der mindestens eine Doppelbindung aufweist. Ein derartiger Heterocyclus kann auch substituiert sein, z.B. durch $C_1$-$C_4$-Alkyl, $C_1$-$C_4$-Alkoxy oder Halogen, insbesondere durch $C_1$-$C_4$-Alkyl. Die Zahl der Alkylgruppen kann dabei 1 bis 6, z.B. 1 bis 5, insbesondere 1 bis 4 und besonders bevorzugt 1 bis 3 sein; besonders bevorzugt sind hierbei Methylgruppen.

Beispielsweise bilden R und $R_1$ gemeinsam mit dem N-Atom und dem ankondensierten Benzolring einen gegebenenfalls durch 1 bis 5 $C_1$-$C_4$-Alkylgruppen substituierten Dihydroindol-, Tetrahydrochinolin-, Tetrahydrochinoxalin-oder Tetrahydro-1,4-benzoxazinrest, wobei der Dihydroindol- und Tetrahydrochinolinrest bevorzugt sind.

(alk) bedeutet z.B. einen Methylen-, Ethylen-, 1,3-Propylen-, 1,4-Butylen-, 1,5-Pentylen- oder 1,6-Hexylenrest oder deren verzweigte Isomere, wobei diese Reste gegebenenfalls z.B. durch $C_1$-$C_4$-Alkoxy, $C_1$-$C_4$-Alkanoyloxy, $C_1$-$C_4$-Alkoxycarbonyl oder Carbamoyl substituiert sein können. Bevorzugt ist für (alk) die Bedeutung eines $C_2$-$C_4$-Alkylenrestes und insbesondere die Bedeutungen 1,2- oder 1,3-Propylen oder Ethylen, besonders Ethylen.

Bedeuten $R_4$, $R_5$ und/oder $R_6$ einen $C_1$-$C_6$-Alkylrest, so kann dies ein Methyl-, Ethyl-, n- oder iso-Propyl- oder n-, iso-, sec.- oder tert.-Butylrest oder ein geradkettiger oder verzweigter Pentyl- oder Hexylrest sein, der gegebenenfalls z.B. durch Hydroxy, $C_1$-$C_4$-Alkoxy, $C_1$-$C_4$-Alkanoyloxy, $C_1$-$C_4$-Alkoxycarbonyl oder Carbamoyl substituiert ist. Vorzugsweise handelt es sich hierbei um einen unsubstituierten oder durch Hydroxy substituierten $C_1$-$C_4$-Alkylrest und insbesondere um den Methyl-oder Ethylrest.

Bedeuten $R_4$, $R_5$ und $R_6$ jeweils einen Alkylrest, so können diese verschieden oder, vorzugsweise, gleich sein.

Stellen $R_4$, $R_5$ und/oder $R_6$ einen Arylrest dar, so kann dies z.B. ein unsubstituierter oder z.B. durch Hydroxy, $C_1$-$C_4$-Alkyl, $C_1$-$C_4$-Alkoxy, Halogen, $C_1$-$C_4$-Alkanoylamino, $C_1$-$C_4$-Alkylsulfonyl, Sulfamoyl, Nitro, Cyano und/oder Carbamoyl substituierter Phenylrest sein.

$R_4$, $R_5$ und $R_6$ als Arylrest stehen bevorzugt für einen unsubstituierten oder durch Methyl, Methoxy, Chlor oder Acetylamino substituierten Phenylrest.

Stehen $R_4$, $R_5$ und/oder $R_6$ für einen Aralkylrest, so kann dies z.B. ein Benzyl- oder Phenylethylrest, welcher gegebenenfalls z.B. wie zuvor für den Arylrest geschildert substituiert ist, sein.

$R_4$, $R_5$ und $R_6$ bedeuten als Aralkylrest vorzugsweise einen unsubstituierten oder durch Methyl, Methoxy, Chlor oder Acetylamino substituierten Benzylrest und besonders bevorzugt den Benzylrest.

Für $R_8$ und $R_9$ gelten unabhängig voneinander je die zuvor für $R_4$ als gegebenenfalls substituiertes $C_1$-$C_6$-Alkyl angegebenen Bedeutungen und Bevorzugungen.

Die Reste $R_8$ und $R_9$ sind verschieden oder vorzugsweise gleich und bedeuten insbesondere bevorzugt je Methyl oder Ethyl.

Stehen $R_{10}$ und/oder $R_{11}$ für gegebenenfalls substituiertes $C_1$-$C_6$-Alkyl, gegebenenfalls substituiertes $C_7$-$C_{12}$-Alkyl oder gegebenenfalls substituiertes Aryl, so gelten unabhängig die zuvor für $R_4$ angegebenen Bedeutungen und Bevorzugungen.

Die Reste $R_{10}$ und $R_{11}$ sind verschieden oder vorzugsweise gleich und bedeuten insbesondere bevorzugt je Wasserstoff.

Steht das Symbol $-NA^{\oplus}$ für einen aliphatisch heterocyclischen Rest, so kann sich dieser z.B. von einem N-alkylierten Piperidin, Morpholin, Tetrahydropyrrol, Dihydropyrazol oder Oxazolidin ableiten. Bevorzugt steht hierbei die Gruppe $-NA^{\oplus}$ für einen Rest

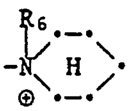 , 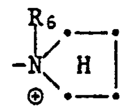 , 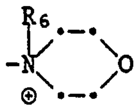 oder 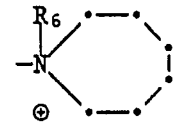 ,

worin für $R_6$ die zuvor genannten Bedeutungen und Bevorzugungen gelten.

Steht das Symbol $-NA^{\oplus}$ für einen aromatisch heterocyclischen Rest, so kann sich dieser z.B. von den folgenden heterocyclischen Verbindungen ableiten: Pyrrol, Indol, Pyrazol, Imidazol, Benzimidazol-, Oxazol, Benzoxazol, Thiazol, Benzthiazol, 1,2,4- oder 1,3,4-Thiadiazol, 1,2,3- oder 1,2,4-Triazol, Benztriazol, Pyridin, Pyrimidin, Pyrazin, Chinolin oder Isochinolin, wobei die genannten Verbindungen gegebenenfalls z.B. durch $C_1$-$C_4$-Alkyl, $C_1$-$C_4$-Alkoxy, Carbamoyl oder Halogen substituiert sein können und kein Wasserstoffatom an einem Ringstickstoffatom gebunden enthalten dürfen; entsprechende Stickstoffatome z.B. im Pyrrol, Indol, Pyrazol, Imidazol und Triazol sind z.B. durch $C_1$-$C_4$-Alkyl oder gegebenenfalls wie zuvor geschildert substituiertes Phenyl substituiert.

Steht die Gruppe $-NA^{\oplus}$ in Formel (1) für einen aromatisch-heterocyclischen Rest, so entspricht dieser vorzugsweise der Formel

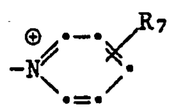

(4)

worin $R_7$ Wasserstoff, Methyl oder Carbamoyl bedeutet; besonders bevorzugt steht die Gruppe $-NA^{\oplus}$ als heterocyclisch-aromatischer Rest für den Pyridinium- oder o-, m- oder p-Methylpyridiniumrest.

$X^{\ominus}$ bedeutet z.B. ein Anion $J^{\ominus}$, $Br^{\ominus}$ und insbesondere $Cl^{\ominus}$.

Das erfindungsgemässe Verfahren ist z.B. geeignet für die Herstellung von Verbindungen der Formel

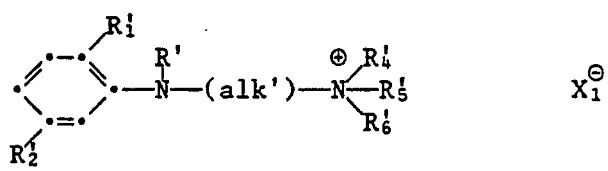

(1a)

und ganz besonders für die Herstellung von Verbindungen der Formel

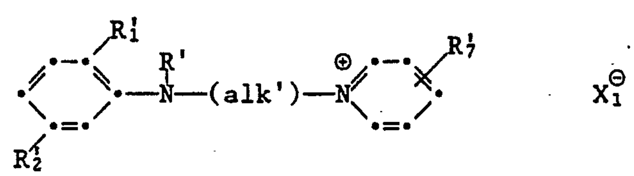

(1b),

worin $R_1'$ und $R_2'$ unabhängig voneinander Wasserstoff, Methyl, Chlor oder Acetylamino bedeuten, $R'$ Wasserstoff oder $C_1$-$C_4$-Alkyl ist, (alk') für einen geradkettigen oder verzweigten $C_2$-$C_4$-Alkylenrest steht, $R_4'$, $R_5'$ und $R_6'$ unabhängig voneinander einen unsubstituierten oder durch Hydroxy substituierten $C_1$-$C_4$-Alkylrest oder einen unsubstituierten oder durch Methyl, Methoxy, Chlor oder Acetylamino substituierten Benzylrest bedeuten, $R_7'$ Wasserstoff oder Methyl ist und $X_1^{\ominus}$ für ein Bromid- und insbesondere für ein Chloridion steht.

Als Halogenierungsmittel kommen für das erfindungsgemässe Verfahren alle üblicherweise für die Halogenierung von Alkylhydroxiden verwendeten Halogenierungs-Reagentien in Frage; im Vordergrund stehen anorganische Säurehalogenide, z.B. solche der Formel $PX_3$, $PX_5$, $POX_3$, $SOX_2$ oder $SO_2X_2$, sowie

organische Säurehalogenide, z.B. solche der Formel
$R_{12}$-$SO_2$-X oder

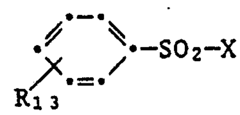
,

worin X jeweils Halogen bedeutet, $R_{12}$ für einen $C_1$-$C_4$-Alkylrest steht und $R_{13}$ z.B. Wasserstoff, $C_1$-$C_4$-Alkyl, $C_1$-$C_4$-Alkoxy, Halogen oder Nitro bedeutet. Bevorzugt sind insbesondere diejenigen obengenannten anorganischen und organischen Halogenierungsmittel, worin X Chlor, $R_{12}$ Methyl oder Ethyl, und $R_{13}$ Wasserstoff, Methyl, Chlor, Brom oder Nitro bedeuten.

Beispiele für geeignete Halogenierungsmittel sind: Phosphortrichlorid ($PCl_3$), Phosphorpentachlorid ($PCl_5$), Phosphoroxychlorid ($POCl_3$), Thionylchlorid ($SOCl_2$), Sulfurylchlorid ($SO_2Cl_2$), Methansulfochlorid, p-Chlor- oder p-Bromphenylsulfochlorid, p-Toluolsulfochlorid, m- oder p-Nitrophenylsulfochlorid, Benzolsulfochlorid sowie die entsprechenden Jod- und Brom-Verbindungen.

Geeignet sind daneben auch Mischungen von mehreren Halogenierungsmitteln, z.B. eine Mischung von $POCl_3$ und Thionylchlorid.

Vorzugsweise verwendet man als Halogenierungsmittel $PCl_3$, $PCl_5$, $POCl_3$, Methansulfochlorid, Benzolsulfochlorid, p-Toluolsulfochlorid, und besonders bevorzugt ist die Verwendung von $POCl_3$.

Das Halogenierungsmittel wird im allgemeinen in solchen Mengen eingesetzt, dass mindestens äquivalente Mengen und vorzugsweise ein gewisser Ueberschuss an Halogenatomen X bzw. Halogenidionen $X^\ominus$, bezogen auf die Verbindung der Formel (3), vorliegen.

Die Menge an Halogenierungsmittel wird vorteilhaft so bemessen, dass sie mindestens 1 Val und vorzugsweise 1 bis 3 Val Halogenatome X bzw. Halogenidionen $X^\ominus$ pro Val Verbindung der Formel (3) enthält.

Beispielsweise verwendet man pro Mol Verbindung der Formel (3) z.B. 0,33 bis 1,0 Mol, vorzugsweise 0,35 bis 0,6 Mol und besonders bervorzugt 0,4 bis 0,5 Mol Verbindung der Formel $PX_3$ oder $POX_3$, worin X jeweils die oben angegebene Bedeutung hat.

Bei den Verbindungen der Formel (2a) handelt es sich um die dem Rest $-NA^\oplus$ zugrunde liegenden ungeladenen Stickstoffverbindungen NA; diese wie auch die Verbindungen der Formel (3) sind an sich bekannt oder können in an sich bekannter Weise erhalten werden.

Die Stickstoffverbindung der Formel (2a) wird im allgemeinen im Ueberschuss, bezogen auf die Verbindung der Formel (3), eingesetzt, wobei das Molverhältnis z.B. 1,5:1 bis 10:1, vorzugsweise 2:1 bis 5:1 und besonders bevorzugt 2:1 bis 3,5:1 beträgt. Möglich sind auch Gemische von verschiedenen Verbindungen der Formel (2a). Schliesslich kann es, insbesondere bei tiefsiedenden Verbindungen der Formel (2a), angebracht sein, zusätzlich zu der Verbindung der Formel (2a) ein inertes Lösungsmittel, z.B. einen aliphatischen oder aromatischen Kohlenwasserstoff oder vorzugsweise ein dipolar aprotisches Lösungsmittel, zu verwenden. Geeignete aliphatische und aromatische Kohlenwasserstoffe sind z.B. geradkettige und verzweigte Alkane bzw. Alkangemische (Petrolether, Benzine), Toluol, o-, m- oder p-Xylol sowie Xylolgemische. Als dipolar aprotische Lösungsmittel kommen z.B. in Betracht: Formamid, N-Methylformamid, N,N-Dimethylformamid, N-Methylpyrrolidon, Dimethylsulfoxid, Sulfolan, Chlorbenzol, 1-Chlornaphthalin, Ethylenglycoldimethylether, Ethylenglycoldiethylether, Diethylenglycoldimethylether. Geeignet sind auch Gemische von verschiedenen Lösungsmitteln. Es ist jedoch bevorzugt, kein zusätzliches Lösungsmittel in das Verfahren einzusetzen.

Das erfindungsgemässe Verfahren wird vorteilhaft ausgeführt, indem man die Hydroxy-Verbindung der Formel (3) und die Stickstoffverbindung der Formel (2a) im Reaktionsgefäss z.B. bei Raumtemperatur vorlegt und das Halogenierungsmittel zulaufen lässt; die Zulaufgeschwindigkeit wird hierbei so geregelt, dass die Temperatur des Reaktionsgemisches einen Wert von z.B. 150 °C, vorzugsweise 140 °C, nicht übersteigt.

Es ist als besonderer Vorteil des erfindungsgemässen Verfahrens anzusehen, dass die Temperaturführung bei dieser exothermen Reaktion aufgrund der Anwesenheit der Stickstoffverbindung der Formel (2a) auch bei grossen Ansätzen unproblematisch ist und ohne besondere Kühlungsmassnahmen, z.B. ausschliesslich mit Luftkühlung, erfolgen kann.

Die Zutropfzeit des Halogenierungsmittels hängt stark von der Ansatzgrösse ab und kann z.B. 15 Minuten bis 5 Stunden, vorzugsweise 20 bis 60 Minuten und insbesondere 25 bis 45 Minuten betragen.

Im Anschluss an die Zugabe des Halogenierungsmittels lässt man das Reaktionsgemisch im allgemeinen bei erhöhter Temperatur, die z.B. 60 bis 200 °C, vorzugsweise 75 bis 150 °C und insbesondere 100 bis 150 °C, betragen kann, ausreagieren; vorzugsweise erhitzt man dazu das Reaktionsgemisch unter Rückfluss, wobei die benötigte Zeit in Abhängigkeit von den Edukten variiert, jedoch im allgemeinen z.B. 2 bis 24 Stunden, vorzugsweise 5 bis 20 Stunden und insbesondere 8 bis 15 Stunden beträgt.

Die gemäss dem Verfahren erhaltenen Verbindungen der Formel (1) werden anschliessend in an sich bekannter Weise isoliert und gereinigt, z.B. indem man die flüssigen organischen Anteile des Reaktionsgemisches destillativ entfernt, das Produkt in Wasser aufnimmt und gegebenenfalls eine Wasserdampfdestillation anschliesst.

Eine bevorzugte Ausführungsform der vorliegenden Erfindung betrifft ein Verfahren zur Herstellung von Verbindungen der zuvor angegebenen Formel (1), worin $R_1$, $R_2$ und $R_3$ unabhängig voneinander Wasserstoff, Methyl, Ethyl, Methoxy, Ethoxy, Chlor, Acetylamino oder Propionylamino bedeuten, R Wasserstoff oder ein $C_1$-$C_6$-Alkylrest ist, (alk) für einen $C_2$-$C_4$-Alkylenrest steht, $-NA^{\oplus}$ a) einen Rest der zuvor angegebenen Formel (2), worin $R_4$, $R_5$ und $R_6$ unabhängig voneinander einen unsubstituierten oder durch Hydroxy substituierten $C_1$-$C_4$-Alkylrest oder einen unsubstituierten oder durch Methyl, Methoxy, Chlor oder Acetylamino substituierten Benzyl-oder Phenylrest bedeuten, b) einen Rest der Formel

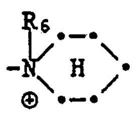 , 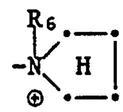 , 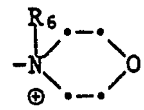 oder 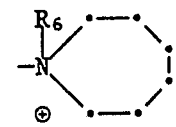 ,

worin $R_6$ $C_1$-$C_4$-Alkyl bedeutet, oder c) einen unsubstituierten oder durch $C_1$-$C_4$-Alkyl, $C_1$-$C_4$-Alkoxy, Carbamoyl oder Halogen substituierten positiv geladenen Pyrrol-, Indol-, Pyrazol-, Imidazol-, Benzimidazol-, Oxazol-, Benzoxazol, Thiazol-, Benzthiazol-, 1,2,4- oder 1,3,4-Thiadiazol-, 1,2,3-oder 1,2,4-Triazol-, Benztriazol-, Pyridin-, Pyrimidin-, Pyrazin-, Chinolin-oder Isochinolinrest darstellt, und $X^{\ominus}$ ein Jodid-, Bromid- oder Chloridion ist, dadurch gekennzeichnet, dass man eine Verbindung der Formel

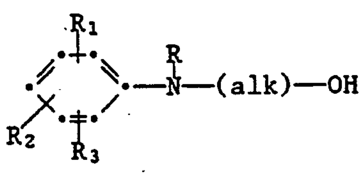 (3)

und eine Verbindung der Formel

NA (2a),

worin die Variablen R, $R_1$, $R_2$, $R_3$ und (alk) jeweils die oben angegebene Bedeutung haben und NA die dem Rest $-NA^{\oplus}$ zugrundeliegende ungeladene Stickstoffverbindung ist, in einem Reaktionsgefäss bei Raumtemperatur vorlegt, ein Halogenierungsmittel der Formel $PX_3$, $PX_5$, $POX_3$, $SOX_2$ oder $SO_2X_2$, worin X Jod, Brom oder Chlor bedeutet, so zulaufen lässt, dass die Temperatur des Reaktionsgemisches 150 °C nicht übersteigt, und das Reaktionsgemisch bei 75 bis 150 °C ausreagieren lässt.

Eine besonders bevorzugte Ausführungsform der vorliegenden Erfindung betrifft ein Verfahren zur Herstellung von Verbindungen der Formel

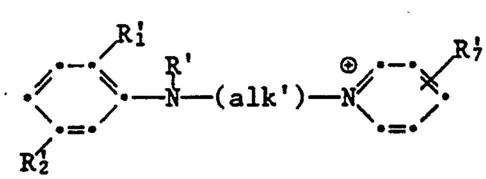 $X_1^{\ominus}$ (1b),

worin $R'$, $R_1'$, $R_2'$, $R_7'$, (alk') und $X_1^{\ominus}$ jeweils die zuvor angegebene Bedeutung haben, dadurch gekennzeichnet, dass man eine Verbindung der Formel

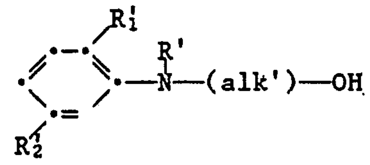

(3a)

und eine Verbindung der Formel

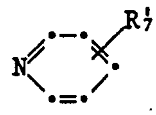

(4a)

in einem Reaktionsgefäss bei Raumtemperatur vorlegt, ein Halogenierungsmittel $POX_3$ so zulaufen lässt, dass die Temperatur des Reaktionsgemisches 150 °C nicht übersteigt und das Reaktionsgemisch bei 100 bis 150 °C ausreagieren lässt, wobei $R'$, $R_1'$, $R_2'$, $R_7'$ und (alk') in den Formeln (3a) und (4a) jeweils die zuvor angegebene Bedeutung haben und X Brom und insbesondere Chlor bedeutet.

Das erfindungsgemässe Eintopf-Verfahren zur Herstellung von kationischen Kupplungskomponenten bietet gegenüber den herkömmlichen 2-Stufen-Verfahren die schon zuvor genannten Vorteile bezüglich der Verfahrenssicherheit, sowie ökonomische und ökologische Vorteile, die sich allesamt aus der Tatsache ergeben, dass man eine Zwischenisolierung einspart und die Edukte im Eintopf-Verfahren zur Reaktion bringt.

Eine Zwischenisolierung verbraucht nämlich Lösungsmittel, Energie, die z.B. für eine Destillation benötigt wird, Zeit und verringert die Gesamtausbeute drastisch. Darüberhinaus kommt man beim erfindungsgemässen Verfahren mit wenig Halogenierungsmittel aus; die Einsparung an Halo genierungsmittel gegenüber den bekannten 2-Stufen-Verfahren beträgt ca. 50 % und mehr. Aus alledem folgt, dass kationische Kupplungskomponenten nach dem erfindungsgemässen Verfahren einfach, sicher und mit hoher Ausbeute, insgesamt also mit maximaler Wirtschaftlichkeit, hergestellt werden können.

Die Verbindungen der Formel (1) stellen wertvolle Zwischenprodukte dar, welche insbesondere als Kupplungskomponenten für die Herstellung von kationischen Farbstoffen in an sich bekannter Weise brauchbar sind. Die erhaltenen Farbstoffe, die an sich bekannt sind, eignen sich zum Färben z.B. von sauer modifizierten Synthesefasern, insbesondere solchen aus Polyacrylnitrilmaterial, und ergeben Färbungen mit hervorragenden Allgemeinechtheiten.

In den folgenden Beispielen sind Teile in Gewichtsteile, Prozente in Gewichtsprozenten und Temperaturen in Grad Celsius angegeben.

Beispiel 1:

In einem Rührkolben werden 165,2 Teile N-Ethyl-N-(2-hydroxyethyl)-anilin und 205,7 Teile Pyridin bei Raumtemperatur vorgelegt und 69 Teile Phosphoroxychlorid ($POCl_3$) zugetropft; dabei steigt die Temperatur auf 130 °C bis 135 °C (Dauer des Zutropfens ca. 30 bis 35 Minuten).

Nach beendetem $POCl_3$-Zulauf wird das Reaktionsgemisch ca. 10 Stunden am Rückfluss erhitzt (Temperatur ca. 130 bis 135 °C). Anschliessend wird das überschüssige Pyridin im leichten Vakuum abdestilliert und das erhaltene rohe Produkt der Formel

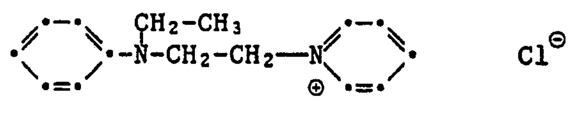

in üblicher Weise aufgearbeitet oder mit Wasser verdünnt und direkt zum entsprechenden Farbstoff weiterverarbeitet; die Ausbeute an kationischer Kupplungskomponente ist nahezu quantitativ und die Raum-Zeit-Ausbeute hervorragend.

Vergleichsbeispiel 1:

Führt man die im Beispiel 1 beschriebene Umsetzung nach einem 2-Stufen-Verfahren aus, indem man zunächst N-Ethyl-N-(2-hydroxyethyl)-anilin mit $POCl_3$ (ca. doppelte Menge verglichen mit Beispiel 1 notwendig, um die Rührbarkeit des Gemisches zu gewährleisten) reagieren lässt, die dabei erhaltene Chlorethyl-Verbindung isoliert und anschliessend mit Pyridin umsetzt, erhält man dieselbe Pyridinium-Verbindung lediglich mit einer gegenüber dem Verfahren gemäss Beispiel 1 halbierten Raum-Zeit-Ausbeute, d.h. gleich grosse Reaktionsgefässe vorausgesetzt erhält man nur halb so viel Pyridinium-Verbindung pro Zeiteinheit wie bei Verwendung des Verfahrens gemäss Beispiel 1.

Beispiel 1a:

In einem Rührkolben werden 165,2 Teile N-Ethyl-N-(2-hydroxyethyl)-anilin bei Raumtemperatur vorgelegt; dazu wird eine Lösung von 62,5 Teilen Phosphorpentachlorid ($PCl_5$) in 600 Teilen Pyridin innerhalb von ca. 30 Minuten zugetropft, wobei die Temperatur auf ca. 70 °C ansteigt. Anschliessend wird das Gemisch 1 Stunde auf 100 °C erhitzt. Man destilliert dann im leichten Vakuum unter stetiger Temperaturerhöhung 300 Teile Pyridin ab und erhitzt das Reaktionsgemisch anschliessend ca. 10 Stunden am Rückfluss (Temperatur ca. 130-135 °C). Die Aufarbeitung erfolgt analog zu Beispiel 1 und ergibt die identische Pyridinium-Verbindung mit vergleichbarer Raum-Zeit-Ausbeute.

Beispiel 1b:

In einem Rührkolben werden 165,2 Teile N-Ethyl-N-(2-hydroxyethyl)-anilin und 261 Teile Pyridin bei Raumtemperatur vorgelegt und innerhalb von ca. 30 Minuten 126 Teile Methansulfochlorid zugetropft; die Temperatur steigt dabei auf 90 bis 95 °C an. Nach beendetem Methansulfochlorid-Zulauf wird das Reaktionsgemisch ca. 10 Stunden am Rückfluss erhitzt (Temperatur ca. 130-135 °C). Die Aufarbeitung erfolgt analog zu Beispiel 1 und ergibt die identische Pyridinium-Verbindung mit vergleichbarer Raum-Zeit-Ausbeute.

Beispiel 1c:

In einem Rührkolben werden 165,2 Teile N-Ethyl-N-(2-hydroxyethyl)-anilin und 261 Teile Pyridin bei Raumtemperatur vorgelegt und in 30 Minuten 140,5 Teile Benzolsulfochlorid zugetropft. Die Temperatur steigt auf 100 °C. Nach beendetem Benzolsulfochlorid-Zulauf wird das Reaktionsgemisch ca. 10 Stunden am Rückfluss erhitzt (Temperatur ca. 130-135 °C). Die Aufarbeitung erfolgt analog zu Beispiel 1 und ergibt die identische Pyridinium-Verbindung mit vergleichbarer Raum-Zeit-Ausbeute.

Beispiel 1d:

In einem Rührkolben werden 165,2 Teile N-Ethyl-N-(2-hydroxyethyl)-anilin und 261 Teile Pyridin bei 30 °C vorgelegt und innerhalb 30 Minuten 68,7 Teile Phosphortrichlorid ($PCl_3$) zugetropft. Die Temperatur steigt auf 100 °C. Nach beendetem $PCl_3$-Zulauf wird das Reaktionsgemisch ca. 10 Stunden am Rückfluss erhitzt (Temperatur ca. 130-135 °C). Die Aufarbeitung erfolgt analog zu Beispiel 1 und ergibt die identische Pyridinium-Verbindung mit vergleichbarer Raum-Zeit-Ausbeute.

Beispiel 1e:

In einem Rührkolben werden 165,2 Teile N-Ethyl-N-(2-hydroxyethyl)-anilin und 300 Teile Dimethylformamid bei Raumtemperatur vorgelegt und innerhalb 30 Minuten 68,7 Teile Phosphortrichlorid ($PCl_3$) zugetropft. Die Temperatur steigt auf 70 bis 75 °C. Nach zweistündigem Ausrühren bei 80-90 °C werden 261 Teile Pyridin zugegeben und das Gemisch ca. 10 Stunden lang auf 100-110 °C erhitzt. Die Aufarbeitung erfolgt analog zu Beispiel 1 und ergibt die identische Pyridiniumverbindung mit vergleichbarer Raum-Zeit-Ausbeute.

Beispiele 2-16:

Verfährt man wie im Beispiel 1 beschrieben, verwendet jedoch äquivalente Mengen der in der Tabelle angegebenen Hydroxyalkylverbindungen und Stickstoffverbindungen, so erhält man analoge kationische Kupplungskomponenten in jeweils vergleichbarer Ausbeute.

| Beispiel Nr. | Hydroxyalkylverbindung | Stickstoffverbindung |
|---|---|---|
| 2 | N-Ethyl-N-(2-hydroxyethyl)-anilin | p-Methylpyridin |
| 3 | N-Ethyl-N-(2-hydroxyethyl)-anilin | m-Methylpyridin |
| 4 | N-Ethyl-N-(2-hydroxyethyl)-anilin | o-Methylpyridin |
| 5 | N-Methyl-N-(2-hydroxyethyl)-anilin | Pyridin |
| 6 | 2-Chlor-N-methyl-N-(2-hydroxyethyl)-anilin | p-Methylpyridin |
| 7 | 3-Chlor-N-methyl-N-(2-hydroxyethyl)-anilin | p-Methylpyridin |
| 8 | 2,5-Dichlor-N-methyl-N-(2-hydroxyethyl)-anilin | p-Methylpyridin |
| 9 | 2-Chlor-N-(2-hydroxyethyl)-anilin | Pyridin |
| 10 | 2-Chlor-N-(2-hydroxyethyl)-anilin | p-Methylpyridin |
| 11 | 3-Methyl-N-ethyl-N-(2-hydroxyethyl)-anilin | Pyridin |
| 12 | N,N-Di-(2-hydroxyethyl)-anilin | Pyridin |
| 13 | 2-Chlor-N-(2-hydroxyethyl)-anilin | p-Methylpyridin |
| 14 | N-Ethyl-N-(2-hydroxyethyl)-anilin | 1-Dimethylamino-2-propanol |
| 15 | N-Ethyl-N-(2-hydroxyethyl)-anilin | N-Methylmorpholin |
| 16 | N-Ethyl-N-(2-hydroxyethyl)-anilin | N,N-Dimethylhydrazin |

## Ansprüche

1. Verfahren zur Herstellung von Verbindungen der Formel

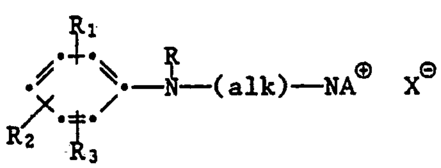

(1)

worin $R_1$, $R_2$ und $R_3$ unabhängig voneinander Wasserstoff, $C_1$-$C_4$-Alkyl, $C_1$-$C_4$-Alkoxy, Halogen oder $C_1$-$C_4$-Alkanoylamino bedeuten,
R Wasserstoff, gegebenenfalls substituiertes $C_1$-$C_{12}$-Alkyl oder ein Rest -(alk)-$NA^{\oplus}X^{\ominus}$ ist oder
R zusammen mit $R_1$ in ortho-Stellung zur Aminogruppe einen teilhydrierten, gegebenenfalls substituierten 5- bis 7-gliedrigen heterocyclischen Ring, der gegebenenfalls noch zusätzliche N-, S- oder O-Atome als Ringglieder enthalten kann, bilden, (alk) einen gegebenenfalls substituierten $C_1$-$C_6$-Alkylenrest bedeutet,
-$NA^{\oplus}$ für den Rest einer Aminoverbindung der Formel

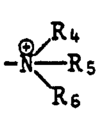

(2) oder

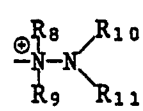

(2'),

worin $R_4$, $R_5$ und $R_6$ unabhängig voneinander gegebenenfalls substituiertes $C_1$-$C_6$-Alkyl, gegebenenfalls substituiertes $C_7$-$C_{12}$-Aralkyl oder gegebenenfalls substituiertes Aryl bedeuten, $R_8$ und $R_9$ unabhängig voneinander je gegebenenfalls substituiertes $C_1$-$C_6$-Alkyl sind und $R_{10}$ und $R_{11}$ unabhängig voneinander je für Wasserstoff, gegebenenfalls substituiertes $C_1$-$C_6$-Alkyl, gegebenenfalls substituiertes $C_7$-$C_{12}$-Aralkyl oder gegebenenfalls substituiertes Aryl stehen, oder für den positiv geladenen Rest einer 5-7 gliedrigen aliphatisch-heterocyclischen oder aromatisch-heterocyclischen Verbindung, die gegebenenfalls noch zusätzliche N-, S- oder O-Atome als Ringglieder enthält, steht, und $X^{\ominus}$ ein Halogenidion bedeutet, dadurch gekennzeichnet, dass man eine Verbindung der Formel

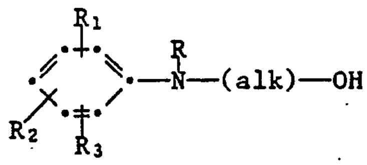

(3)

mit einem Halogenierungsmittel, in Gegenwart einer Verbindung der Formel
NA (2a)
unter Freisetzung von Anionen $X^{\ominus}$
umsetzt, wobei R, $R_1$, $R_2$, $R_3$ und (alk) in der Formel (3) jeweils die oben angegebene Bedeutung haben und NA die dem Rest $-NA^{\oplus}$ zugrunde liegende ungeladene Stickstoffverbindung darstellt.

2. Verfahren gemäss Anspruch 1, dadurch gekennzeichnet, dass $R_1$, $R_2$ und $R_3$ unabhängig voneinander Wasserstoff, Methyl, Ethyl, Methoxy, Ethoxy, Chlor, Acetylamino oder Propionylamino bedeuten.

3. Verfahren gemäss Anspruch 1 oder 2, dadurch gekennzeichnet, dass einer der Reste $R_1$, $R_2$ und $R_3$ Wasserstoff und die anderen beiden Reste unabhängig voneinander Wasserstoff, Methyl, Chlor oder Acetylamino bedeuten.

4. Verfahren gemäss einem der Ansprüche 1 bis 3, dadurch gekennzeichnet, dass R Wasserstoff oder $C_1$-$C_6$-Alkyl, vorzugsweise Wasserstoff oder $C_1$-$C_4$-Alkyl und insbesondere Wasserstoff, Methyl oder Ethyl bedeutet.

5. Verfahren gemäss einem der Ansprüche 1 bis 4, dadurch gekennzeichnet, dass (alk) für einen $C_2$-$C_4$-Alkylenrest, vorzugsweise für 1,2- oder 1,3-Propylen oder Ethylen, steht.

6. Verfahren gemäss einem der Ansprüche 1 bis 5, dadurch gekennzeichnet, dass $-NA^{\oplus}$ a) einen Rest der im Anspruch 1 angegebenen Formel (2), worin $R_4$, $R_5$ und $R_6$ unabhängig voneinander einen unsubstituierten oder durch Hydroxy substituierten $C_1$-$C_4$-Alkylrest oder einen unsubstituierten oder durch Methyl, Methoxy, Chlor oder Acetylamino substituierten Benzyl- oder Phenylrest bedeuten, b) einen Rest der Formel

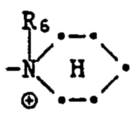

,

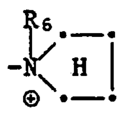

,

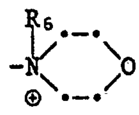

oder

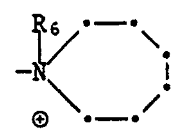

,

worin $R_6$ $C_1$-$C_4$-Alkyl bedeutet, oder c) einen unsubstituierten oder durch $C_1$-$C_4$-Alkyl, $C_1$-$C_4$-Alkoxy, Carbamoyl oder Halogen substituierten positiv geladenen Pyrrol-, Indol-, Pyrazol-, Imidazol-, Benzimidazol-, Oxazol-, Benzoxazol-, Thiazol-, Benzthiazol-, 1,2,4- oder 1,3,4-Thiadiazol, 1,2,3-oder 1,2,4-Triazol-, Benztriazol-, Pyridin-, Pyrimidin-, Pyrazin-, Chinolin-oder Isochinolinrest bedeutet.

7. Verfahren gemäss einem der Ansprüche 1 bis 6, dadurch gekennzeichnet, dass $-NA^{\oplus}$ für einen Rest der im Anspruch 1 angegebenen Formel (2), worin $R_4$, $R_5$ und $R_6$ unabhängig voneinander ein unsubstituierter oder durch Hydroxy substituierter $C_1$-$C_4$-Alkylrest oder ein unsubstituierter oder durch Methyl, Methoxy, Chlor oder Acetylamino substituierter Benzylrest sind, oder für einen Rest der Formel

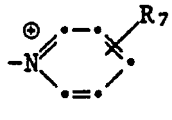

(4),

worin $R_7$ Wasserstoff, Methyl oder Carbamoyl bedeutet, steht.

8. Verfahren gemäss einem der Ansprüche 1 bis 7, dadurch gekennzeichnet, dass $-NA^{\oplus}$ für den Pyridinium- oder o-, m- oder p-Methylpyridiniumrest steht.

9. Verfahren gemäss einem der Ansprüch 1 bis 8, dadurch gekennzeichnet, dass $X^{\ominus}$ für das Chloridion $Cl^{\ominus}$ steht.

10. Verfahren gemäss einem der Ansprüche 1 bis 8, dadurch gekennzeichnet, dass man als Halogenierungsmittel ein anorganisches Säurehalogenid $PX_3$, $PX_5$, $POX_3$, $SOX_2$ oder $SO_2X_2$ oder ein organisches Säurehalogenid der Formel
$R_{12}$-$SO_2$-X oder

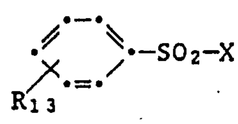 ,

worin X Halogen bedeutet, $R_{12}$ $C_1$-$C_4$-Alkyl ist und $R_{13}$ für Wasserstoff, $C_1$-$C_4$-Alkyl, $C_1$-$C_4$-Alkoxy, Halogen oder Nitro steht, verwendet.

11. Verfahren gemäss einem der Ansprüche 1 bis 10, dadurch gekennzeichnet, dass man als Halogenierungsmittel $PCl_3$, $PCl_5$, $POCl_3$, Methansulfochlorid, Benzolsulfochlorid, p-Toluolsulfochlorid, besonders $POCl_3$, verwendet.

12. Verfahren gemäss einem der Ansprüche 1 bis 11, dadurch gekennzeichnet, dass man die Verbindungen der Formeln (2a) und (3) im Reaktionsgefäss vorlegt, das Halogenierungsmittel zulaufen lässt und das Reaktionsgemisch anschliessend ausreagieren lässt.

13. Verfahren gemäss Anspruch 12, dadurch gekennzeichnet, dass man die Verbindungen der Formeln (2a) und (3) im Molverhältnis 2:1 bis 5:1, vorzugsweise 2:1 bis 3,5:1, bei Raumtemperatur einsetzt.

14. Verfahren gemäss Anspruch 12 oder 13, dadurch gekennzeichnet, dass man das Halogenierungsmittel so zulaufen lässt, dass die Temperatur des Reaktionsgemisches 150 °C nicht übersteigt.

15. Verfahren gemäss einem der Ansprüche 12 bis 14, dadurch gekennzeichnet, dass man das Reaktionsgemisch nach Zugabe des Halogenierungsmittels bei 75 bis 150 °C, vorzugsweise 100 bis 150 °C, ausreagieren lässt.

16. Verfahren zur Herstellung von Verbindungen der Formel (1) gemäss Anspruch 1, worin $R_1$, $R_2$ und $R_3$ unabhängig voneinander Wasserstoff, Methyl, Ethyl, Methoxy, Ethoxy, Chlor, Acetylamino oder Propionylamino bedeuten, R Wasserstoff oder ein $C_1$-$C_6$-Alkylrest ist, (alk) für einen $C_2$-$C_4$-Alkylenrest steht, $-NA^{\oplus}$ a) einen Rest der im Anspruch 1 angegebenen Formel (2), worin $R_4$, $R_5$ und $R_6$ unabhängig voneinander einen unsubstituierten oder durch Hydroxy substituierten $C_1$-$C_4$-Alkylrest oder einen unsubstituierten oder durch Methyl, Methoxy, Chlor oder Acetylamino substituierten Benzyl- oder Phenylrest bedeuten, b) einen Rest der Formel

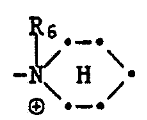 , 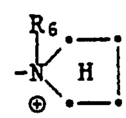 , 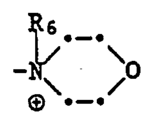 oder 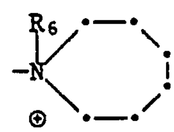 ,

worin $R_6$ $C_1$-$C_4$-Alkyl bedeutet, oder c) einen unsubstituierten oder durch $C_1$-$C_4$-Alkyl, $C_1$-$C_4$-Alkoxy, Carbamoyl oder Halogen substituierten positiv geladenen Pyrrol-, Indol-, Pyrazol-, Imidazol-, Benzimidazol-, Oxazol-, Benzoxazol-, Thiazol-, Benzthiazol-, 1,2,4- oder 1,3,4-Thiadiazol-, 1,2,3- oder 1,2,4-Triazol-, Benztriazol-, Pyridin-, Pyrimidin-, Pyrazin-, Chinolin-oder Isochinolinrest darstellt, und $X^{\ominus}$ ein Jodid-, Bromid- oder Chloridion ist, dadurch gekennzeichnet, dass man eine Verbindung der Formel

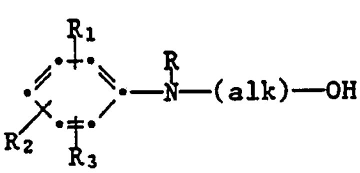 (3)

und eine Verbindung der Formel
NA (2a),

worin die Variablen R, $R_1$, $R_2$, $R_3$ und (alk) jeweils die oben angegebene Bedeutung haben und NA die dem Rest $-NA^{\oplus}$ zugrundeliegende ungeladene Stickstoffverbindung ist, in einem Reaktionsgefäss bei Raumtemperatur vorlegt, ein Halogenierungsmittel der Formel $PX_3$, $PX_5$, $POX_3$, $SOX_2$ oder $SO_2X_2$, worin X Jod, Brom oder Chlor bedeutet, so zulaufen lässt, dass die Temperatur des Reaktionsgemisches 150 °C nicht übersteigt, und das Reaktionsgemisch bei 75 bis 150 °C ausreagieren lässt.

17. Verfahren zur Herstellung von Verbindungen der Formel

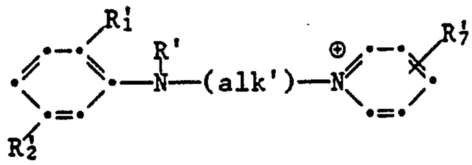 $X_1^{\ominus}$ (1b),

worin $R_1'$ und $R_2'$ unabhängig voneinander Wasserstoff, Methyl, Chlor oder Acetylamino bedeuten, $R'$ Wasserstoff oder $C_1$-$C_4$-Alkyl ist, (alk′) für einen geradkettigen oder verzweigten $C_2$-$C_4$-Alkylenrest steht, $R_7'$ Wasserstoff oder Methyl ist und $X_1^{\ominus}$ für das Bromid- und insbesondere für das Chloridion steht, dadurch gekennzeichnet, dass man eine Verbindung der Formel

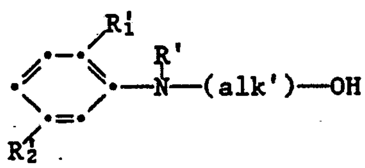 (3a)

und eine Verbindung der Formel

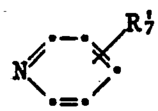 (4a)

in einem Reaktionsgefäss bei Raumtemperatur vorlegt, ein Halogenierungsmittel $POX_3$ so zulaufen lässt, dass die Temperatur des Reaktionsgemisches 150 °C nicht übersteigt und das Reaktionsgemisch bei 100 bis 150 °C ausreagieren lässt, wobei $R'$, $R_1'$, $R_2'$, $R_7'$, (alk′) und $X_1^{\ominus}$ die oben angegebene Bedeutung haben und X Brom und insbesondere Chlor bedeutet.

18. Verwendung der gemäss Anspruch 1 hergestellten Verbindungen der Formel (1) als Kupplungskomponenten für die Herstellung von kationischen Farbstoffen.